Europäisches Patentamt

European Patent Office

Office européen des brevets

(19)

(11) Veröffentlichungsnummer: **0 244 784**

**A2**

(12) **EUROPÄISCHE PATENTANMELDUNG**

(21) Anmeldenummer: **87106343.4**

(22) Anmeldetag: **02.05.87**

(51) Int. Cl.4: **A61N 1/42**

(30) Priorität: **09.05.86 CH 1898/86**

(43) Veröffentlichungstag der Anmeldung:
**11.11.87 Patentblatt 87/46**

(84) Benannte Vertragsstaaten:
**AT BE.CH DE FR GB IT LI LU NL**

(71) Anmelder: **Rosenthal, Moshe, Dr.**
**Steinengraben 67**
**CH-4051 Basel(CH)**

Anmelder: **Savini, Carlo**
**Bruderholzallee 206**
**CH-4059 Basel(CH)**

(72) Erfinder: **Rosenthal, Moshe, Dr.**
**Steinengraben 67**
**CH-4051 Basel(CH)**
Erfinder: **Savini, Carlo**
**Bruderholzallee 206**
**CH-4059 Basel(CH)**

(54) **Elektrotherapeutische Vorrichtung.**

(57) Elektrotherapeutisches Magnetfeld-Heilgerät zur Erzielung einer Heilwirkung während normaler Tätigkeiten eines Patienten. Das Gerät enthält eine Anzahl von Spulen, die auf einem Träger verteilt angeordnet sind, und ein Stromversorgungsgerät, das den Spulen einen Strom mit niederfrequent wechselnder Stromstärke zuführt. Die Spulen sind so ausgelegt, dass sie eine magnetische Feldstärke von 2 - 20 Gauss liefern.

Fig.1

EP 0 244 784 A2

## Elektrotherapeutische Vorrichtung

Die Erfindung betrifft eine elektrotherapeutische Vorrichtung mit einer Anzahl von auf einem Träger angeordneten Spulen und einer Stromversorgungseinrichtung für die Spulen zur Erzeugung eines Magnetfeldes.

Veränderliche magnetische Felder haben einen direkten Einfluss auf lebende Zellen und Zellsysteme, der darauf zu beruhen scheint, dass die im Kolloidalsystem und in den Zellen selbst vorhandenen Ionen zu Bewegungen gezwungen werden. Es ist seit langem bekannt, magnetische Wechselfelder zu therapeutischen Zwecken einzusetzen. Die hierfür verwendeten Felder liegen üblicherweise zwischen 1 und 100 Gauss. Das Magnetfeld der Erde hat im Vergleich dazu ca. ½ Gauss. Die Schwingungsfrequenz der Felder, die zu therapeutischen Zwecken eingesetzt werden, ist in der Regel niedrig, d.h. bis zu einigen hundert Hz .

Aus GB - A - 134 640 ist beispielsweise ein Stuhl für elektro-magnetische Behandlung bekannt. Im Inneren des Stuhls sind an verschiedenen Stellen Elektromagnete angeordnet, von denen Polschuhe in die Nähe der zu behandelnden Körperteile des Patienten führen. Der Stuhl ist derart aufgebaut, dass er sich nur für spezielle Therapiesitzungen eignet. Eine die normale Tätigkeit des Patienten nicht störende Behandlung ist nicht möglich.

FR - A - 716 954 beschreibt eine therapeutische Vorrichtung, die aus einem mit Heiz-und Magnetspulen versehenen Polsterelement besteht. Die Spulen sind in Serie geschaltet und von einem Strom durchflossen, der im Prinzip konstant ist und demzufolge in den Magnetspulen ein konstantes Magnetfeld erzeugt. Der Stromfluss wird lediglich in Abständen durch einen den Wärmespulen zugeordneten Thermostaten vorübergehend unterbrochen.

Aus FR - A - 1 371 236 ist eine schlafbegünstigende Vorrichtung zur Erzeugung eines Magnetfeldes bekannt. Der Vorrichtung liegt die Idee zugrunde, das Erdfeld überzukompensieren in Fällen, in denen seine Richtung nicht dem physiologischen Optimum entspricht. Dementsprechend ist das Magnetfeld in seiner Grösse und seiner Richtung variabel zwecks Anpassung an die Gegebenheiten.

Der vorliegenden Erfindung liegt die Aufgabe zugrunde, ein elektrotherapeutisches Magnetfeld-Heilgerät vorzuschlagen, mit dem eine die normale Tätigkeit des Patienten nicht störend beeinflussende magneto-therapeutische Wirkung in bestimmten Körperzonen erzielt werden kann.

Diese Aufgabe wird erfindungsgemäss dadurch gelöst, dass in einer Vorrichtung der eingangs genannten Art das Stromversorgungsgerät den Spulen einen Strom mit niederfrequent wechselnder Stromstärke zuführt und die Spulen so ausgelegt sind, dass sie eine magnetische Feldstärke von 2 bis 20 Gauss liefern.

Im folgenden wird anhand der beiliegenden Zeichnungen ein Ausführungsbeispiel der Erfindung beschrieben. Es zeigen

Fig.1 eine schematische Darstellung einer bevorzugten Ausführungsform der Erfindung

Fig.2 eine Darstellung einer Feldmessungsaufzeichnung

Fig.3 ein Schaltbild einer für die Vorrichtung nach der Fig.1 geeigneten Steuerschaltung

Fig.4 - 6 schematisch dargestellte Beispiele des Einsatzes der erfindungsgemässen Vorrichtung

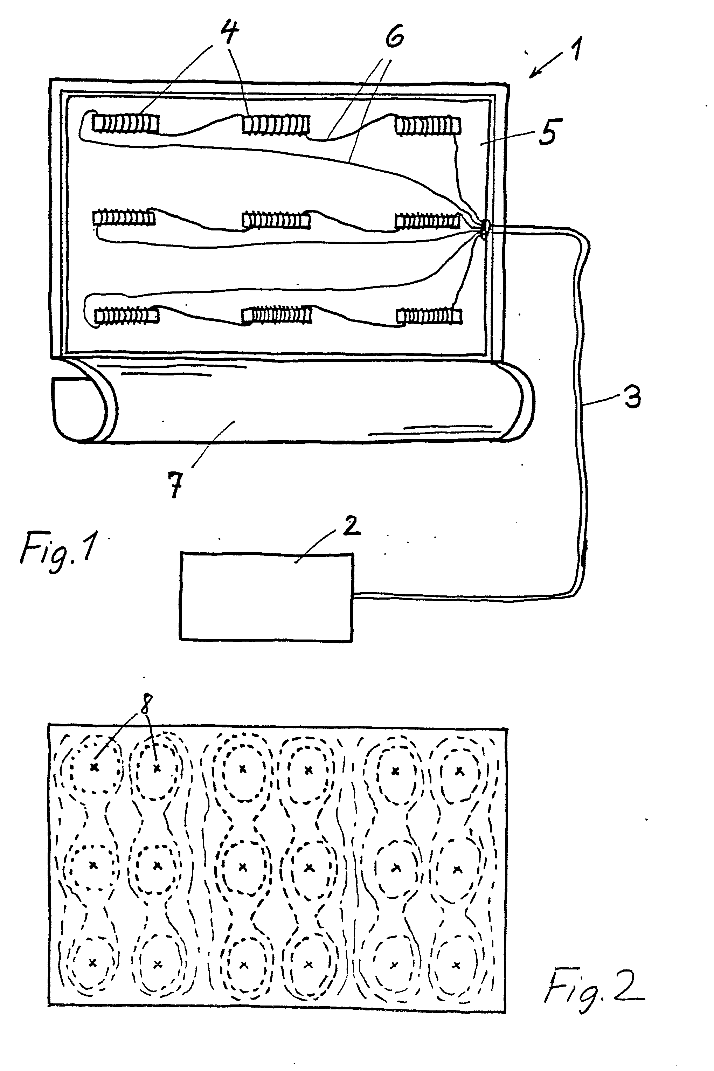

Die in Fig.1 gezeigte Vorrichtung besteht im wesentaus einer Spulenanordnung 1 und einem Stromversorgungsteil 2, die miteinander durch ein Kabel 3 verbunden sind.

Die Spulenanordnung 1 besteht aus einer Anzahl von Spulen 4, im vorliegenden Fall neun, die etwa gleichmässig auf einem flachen Träger 5 verteilt und befestigt sind. Der Träger 5 besteht aus einem flexiblen Material, das aber doch eine gewisse Steifigkeit oder Formstabilität aufweist, um die flächige Verteilung und die Abstände der Spulen voneinander im wesentlichen zu gewährleisten. Ein textiles Gewebe aus einem verhältnismässig steifen Grundmaterial, z.B. grobes Leinen etc., hat sich als geeignet erwiesen.

Die Spulen 4 und die Zuleitungen 6 zu denselben, die am Rand des Trägers 5 zum Kabel 3 vereinigt sind, sind mit einigen grossen Stichen am Träger angeheftet. Alternativ wäre denkbar, am Träger Taschen zur Aufnahme der Spulen vorzusehen. Im Falle eines Trägers aus Kunststoff wäre auch ein Eingiessen oder Einschweissen zwischen zwei Schichten des Trägers möglich.

Die Spulen 4 bestehen aus 300 Windungen eines 0,2 mm starken Kupferdrahts auf einem Weicheisenkern mit den Abmessungen 3 x 15 x 60 mm. Selbstverständlich gelten diese Angaben nur für das vorliegende Ausführungsbeispiel. Andere Abmessungen und Windungszahlen sind ohne weiteres möglich, ohne dass ein grundsätzlich anderes Ergebnis erzielt wird. Auf der anderen Seite kann durch Modifikationen der Abmessungen und der Windungszahlen, sowie durch Aenderungen der Zahl und der Anordnung der Spulen das wirksame Magnetfeld durchaus verändert und angepasst werden, wenn dies erwünscht ist. So können z.B. aus physiologischen Gründen höhere oder niedrigere Feldstärken oder andere Feldverteilungen benötigt werden.

Die Spulen sind in drei Gruppen von je drei Stück angeordnet, wobei jeweils die drei einer Gruppe in Serie geschaltet sind.

Der Träger 5 mit den Spulen 4 ist von einer äusseren Umhüllung 7 in Form eines Bezugs umschlossen, die in Fig.1 geöffnet gezeigt ist. Der Umhüllungsbezug weist an seinem umlaufenden Rand ein sog. Klettverschlussband auf, so dass er geöffnet und abgenommen werden kann, wenn beispielsweise eine Wäsche oder Reinigung nötig ist. Anstelle des Klettbandes wäre auch ein Reissverschluss denkbar. Es wäre aber auch möglich,den Bezug fest zu verschliessen, z.B. zuzunähen.

Wenn der Träger aus zwei Kunststoff-Folien besteht, zwischen denen die Spulen eingeschweisst sind oder wenn die Spulen in eine Kunststoffschicht eingegossen sind, wird im Prinzip keine zusätzliche Umhüllung benötigt.

Für manche Anwendungen kann es vorteilhaft sein, wenn die Umhüllung gepolstert ist, so dass praktische eine Art Kissen entsteht. Dadurch ergibt sich gleichzeitig ein zusätzlicher Schutz für die Spulen und die Zuleitungen. Allerdings ist zu beachten, dass bei einer gepolsterten Umhüllung der Abstand von der zu therapierenden Körperzone des Patienten grösser ist, so dass höhere Feldstärken in Betracht gezogen werden müssen.

Fig.2 zeigt eine Aufzeichnung einer Feldmessung, die im Abstand von 5 cm von der Ebene, in der sich die Spulen befinden, vorgenommen wurde. Die mit "x" markierten Stellen 8 entsprechen den Enden der Spulenkerne. An diesen Stellen ist das Feld jeweils am stärksten und beträgt ca. 2,5 -4 Gauss. Zwischen diesen Punkten ist das Feld - schwächer. Die gestrichelten Linien gleicher Feldstärke wurden durch Messung bestimmt. Von den Stellen 8 nach aussen entspricht die jeweils erste gestrichelte Linie einer Feldstärke von ca. 1.2 - 1,4 Gauss, die zweite 0,8 - 1,0 Gauss. Das magnetische Feld hat also über die gesamte Fläche einen Wert von mindestens 0,5 Gauss und liegt damit über dem Erdfeld und im Bereich der therapeutischen Wirksamkeit. Das Feld ist bewusst verhältnismässig schwach ausgelegt, weil die erfindungsgemässe Vorrichtung für lang andauernde Anwendung konzipiert ist. Dies wird aus physiologischen Gründen als vorteilhaft gegenüber der kurzzeitigen Anwendung hoher Felder erachtet.

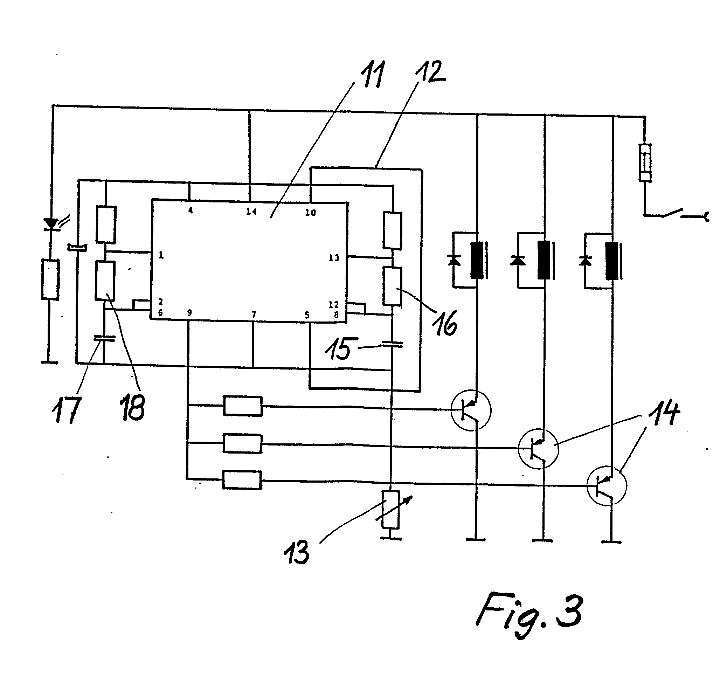

Der Stromversorgungsteil 2 liefert den Strom, der die Spulen 4 zur Erzeugung des Magnetfeldes durchfliesst. Wie bereits erwähnt, wird den Spulen ein Strom mit niederfrequent wechselnder Stromstärke, d.h. im vorliegenden Fall eine niederfrequente Impulsfolge zugeführt, wodurch ein in seiner Stärke wechselndes Magnetfeld erzeugt wird. Der Aufbau des Stromversorgungsteils 2 ist in Fig.3 gezeigt. Die drei in Serie geschalteten Spulen einer Gruppe sind in diesem Diagramm jeweils durch eine einzige Spule 4 repräsentiert.

Ein unter der Bezeichnung "Dual Timer" erhältlicher Impulsgenerator 11 erzeugt zwei Folgen von Impulsen und zwar eine Folge mit niedriger Impulsrate und langer Impulsdauer und eine Folge mit hoher Impulsrate und kurzer Impulsdauer. Diese Impulsfolgen, die an den Ausgängen "5" und "10" des Timers 11 zur Verfügung stehen, sind über die Leitung 12 miteinander verkoppelt. Ein Potentiometer 13 dient der Amplitudenregelung der Impulse. Das resultierende Signal steht am Anschluss "9" des Timers 11 für Treibertransistoren 14 zur Anregung der Spulen 4 zur Verfügung.

Die Impulse können durch Verändern folgender Werte den physiologischen Anforderungen angepasst werden: Durch einen Kondensator 15 in Serie mit dem Potentiometer 13 ist die höhere Impulsrate und durch einen mit dem Kondensator in Serie geschalteten Widerstand 16 deren Impulsdauer einstellbar. Die tiefere Impulsrate ist durch einen entsprechend angeordneten Kondensator 17 und deren Impulsdauer durch einen Widerstand 18 einstellbar.

Fig.4 zeigt eine perspektivische Darstellung eines Automobilsitzes mit Sitzpolster 21, Rückenlehne 22 und Kopf-oder Nackenstütze 23.Letztere soll für den Zweck dieser Beschreibung ebenfalls unter den Begriff "Lehne" fallen. Im vorderen Teil weist das Sitzpolster 1 einen etwas erhöhten Wulst 24 auf, der der Unterstützung der Oberschenkel des Fahrers dient. In der Zeichnung ist im Bereich des Wulstes ein Teil der obersten Polsterschicht und des Bezugs herausgeschnitten. In diesem Ausschnitt ist ein Elektrotherapiegerät 25 nach der vorliegenden Erfindung sichtbar. Es ist so im Sitzpolster angeordnet, dass es sich quer zu den Oberschenkeln des Fahrers erstreckt und sich möglichst nahe bei dessen Kniebereich befindet. Im eingeschalteten Zustand gibt es magnetische Energie in den Kniebereich des Benutzers ab.

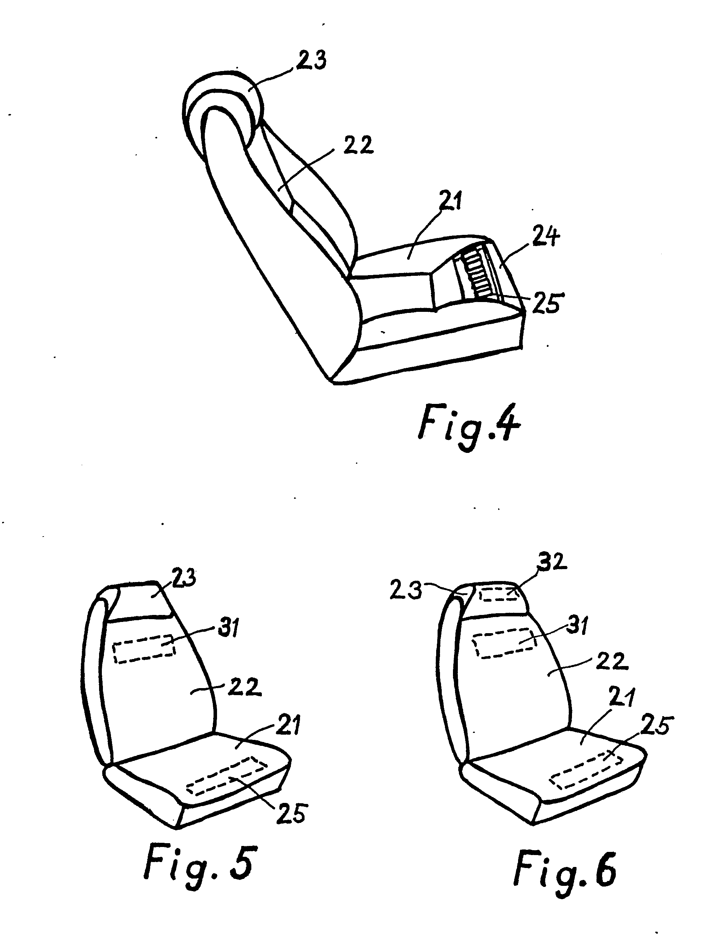

Fig.5 zeigt eine Variante eines Automobilsitzes, in dem zusätzlich eine elektrotherapeutische Vorrichtung 31 in der Rückenlehne 22 im Bereich des Schultergürtels des Benutzers vorgesehen ist. Die Position der Vorrichtungen ist gestrichelt gezeichnet.

Fig.6 zeigt eine weitere Variante, bei der zusätzlich noch ein drittes Elektrotherapiegerät 32 in der Kopf-oder Nackenstütze vorgesehen ist. Vor allem bei Automobilsitzen ist diese Position mit Einwirkung auf den Hinterkopf des Benutzers vorteilhaft, da auf diese Weise der Ermüdung entgegengewirkt wird, die auf die Wirkung der Fahrzeugkarrosserie als Faradayscher Käfig zurückzuführen ist.

**Ansprüche**

1. Elektrotherapeutische Vorrichtung mit einer Anzahl von auf einem Träger (5) angeordneten Spulen (4) und einer Stromversorgungseinrichtung (2) für die Spulen zur Erzeugung eines Magnetfeldes, dadurch gekennzeichnet, dass das Stromversorgungsgerät (2) den Spulen (4) einen Strom mit niederfrequent wechselnder Stromstärke zuführt und die Spulen (4) so ausgelegt sind, dass sie eine magnetische Feldstärke von 2 - 20 Gauss liefern.

2. Elektrotherapeutische Vorrichtung nach Anspruch 1,
dadurch gekennzeichnet, dass die Stromversorgungseinrichtung eine niederfrequente Folge von Stromimpulsen liefert.

3. Elektrotherapeutische Vorrichtung nach Anspruch 1,
dadurch gekennzeichnet, dass der Träger (5) aus einem flexiblen Material besteht.

4. Elektrotherapeutische Vorrichtung nach Anspruch 1,
dadurch gekennzeichnet, dass eine abnehmbare Umhüllung vorhanden ist.

5. Elektrotherapeutische Vorrichtung nach Anspruch 4,
dadurch gekennzeichnet, dass die Umhüllung gepolstert ist.

Fig.1

Fig.2

Fig. 3

Fig.4

Fig. 5

Fig.6